(19) Europäisches Patentamt
European Patent Office
Office européen des brevets

(11) **EP 1 207 448 B1**

(12) **FASCICULE DE BREVET EUROPEEN**

(45) Date de publication et mention
de la délivrance du brevet:
**04.06.2008 Bulletin 2008/23**

(51) Int Cl.:
***G06F 3/00*** *(2006.01)* ***A63B 23/12*** *(2006.01)*

(21) Numéro de dépôt: **01402864.1**

(22) Date de dépôt: **08.11.2001**

(54) **Procédé et système de commande d'un élément à retour d'effort**

Verfahren und System zur Steuerung einer Kraftrückkopplungsvorrichtung

Method and system for controlling a force feedback device

(84) Etats contractants désignés:
**AT BE CH CY DE DK ES FI FR GB GR IE IT LI LU MC NL PT SE TR**

(30) Priorité: **15.11.2000 FR 0014733**

(43) Date de publication de la demande:
**22.05.2002 Bulletin 2002/21**

(73) Titulaire: **FRANCE TELECOM**
**75015 Paris (FR)**

(72) Inventeur: **Hennion, Bernard**
**38140 St. Martin d'Uriage (FR)**

(74) Mandataire: **Loisel, Bertrand et al**
**Cabinet Plasseraud**
**52 rue de la Victoire**
**75440 Paris Cedex 09 (FR)**

(56) Documents cités:
**WO-A-98/06024          FR-A- 2 646 612**

- **SATO M ET AL: "A REMOTE COLLABORATION SYSTEM USING HAND-FORCE VIRTUAL ENVIRONMENT" ELECTRONICS & COMMUNICATIONS IN JAPAN, PART III - FUNDAMENTAL ELECTRONIC SCIENCE,US, SCRIPTA TECHNICA. NEW YORK, vol. 80, no. 4, 1 avril 1997 (1997-04-01), pages 51-58, XP000723464 ISSN: 1042-0967**

**Description**

La présente invention relève du domaine de la transmission de retour d'effort à distance, notamment dans le cadre du développement de mondes virtuels répartis et du développement de moyens de téléprésence.

De la même façon que les techniques de codage de vidéo numérique s'appuient sur les connaissances de la perception visuelle, et les techniques de reconnaissance vocale sur les connaissances de l'ouïe, les techniques haptiques (du grec haptos : la main) s'appuient sur les connaissances des gestes.

Il existe deux sortes de gestes fins réalisables avec la main : les gestes balistiques, comme déplacer la main vers un verre pour le saisir et les gestes avec contre-réactions du toucher, comme celui qui permet après saisie du verre et fermeture de la pince pouce-doigts, de porter le verre à sa bouche. Le cerveau est alors informé en permanence de la force avec laquelle la main enserre le verre, de son poids qui dépend de la quantité de liquide. Le cerveau réagit alors en donnant l'ordre moteur de "pincer" suffisamment ce verre pour qu'il ne tombe pas, mais pas trop toutefois, pour ne pas le casser, ni dépenser une énergie inutile.

Les gestes balistiques activent l'arc moteur mais ils n'activent pas l'arc sensitif en retour du toucher. Le retour d'informations peut être une représentation visuelle de l'espace, mais aussi une carte gestuelle, c'est-à-dire une représentation mentale apprise ou innée câblée dans le cerveau et qui génère automatiquement la séquence des ordres moteurs des muscles de l'épaule, du bras, de la main pour réaliser ce geste balistique en fonction d'une certaine représentation mentale de l'espace, notamment de la distance présumée main-verre.

Pour les gestes balistiques, il est suffisant de transmettre au cerveau les informations sur le geste avec une fréquence d'échantillonnage de 100 Hz. Cela signifie que si on envoie un échantillon du signal toutes les 10 ms, le signal transmis contiendra toute l'information pertinente du geste balistique.

Les gestes avec contre-réactions du toucher activent en même temps l'arc moteur et l'arc sensitif. Le cerveau ferme la boucle et le cycle complet chez l'homme dure moins de 1 ms. La bande passante des neurones sensitifs situés dans les bouts des doigts, c'est-à-dire la fréquence maximale du signal mécanique que ces neurones sont capables de détecter et de transmettre au cerveau, est supérieure à 500 Hz. Si on veut pouvoir coder dans un ordinateur un geste fin, il faut que le système à retour d'efforts utilisé ait lui-même une fréquence de fonctionnement élevée, au moins égale, d'après le théorème de Shannon, au double de la bande passante des doigts.

Dans la pratique, les systèmes à retour d'efforts sur une machine locale fonctionnent à une fréquence typique de 1 KHz en boucle fermée locale, c'est-à-dire qu'une rétroaction est calculée puis exercée sur leurs moteurs puis perçue par la main toutes les 1/1000 de seconde. Cela permet d'éviter l'effet dit de la "brosse à dents électrique" : l'instrument que l'on tient en main ne doit pas donner l'impression de vibrer.

Cette fréquence de 1 KHz résulte du compromis suivant : elle ne doit être ni trop basse pour pouvoir restituer finement l'impression tactile, ni trop haute pour laisser suffisamment de temps à l'ordinateur pour calculer la force de contre-réaction qui va représenter, dans le monde virtuel mécanique, la simulation fine du geste effectué.

Si maintenant on désire transmettre via un réseau de télécommunications, des gestes fins codés par le système à retour d'efforts et des gestes fins avec contre-réactions, le problème se complique du fait de la latence généralement bien supérieure introduite par le réseau lui-même.

Ainsi, la latence en technologie RNIS est de 30 ms, en technologie ADSL de l'ordre de 200 ms, et sur Internet elle peut atteindre 6 s ou même provoquer le rejet pur et simple du message. Sur ADSL et Internet, la latence varie du fait de la nature asynchrone des réseaux. La cadence de 1 KHz est donc beaucoup trop élevée pour pouvoir être maintenue si la boucle fermée inclut un aller-retour via le réseau - le geste est codé puis transmis via le réseau, il est appliqué à un objet distant, la contre-réaction de cet objet est à son tour codée et transmise en retour via le réseau -.

Un geste balistique peut être transmis avec un retard de l'ordre de grandeur de 10 ms. En effet, la vue est un sens monodirectionnel : l'oeil est une sorte de caméra enregistrant une scène et le cerveau, à une tolérance près, peut percevoir avec un léger retard le film visuel précis sans perturber l'exécution du geste.

Au contraire, un geste fin avec contre-réaction nécessite de boucler en moins d'une milliseconde, l'aller-retour de décision de l'intensité de la force à exercer :

- émission de l'ordre au muscle via l'arc sensitif moteur,
- action mécanique de la main sur le verre,
- sensation du toucher du verre (augmentation de la pression de contact) au niveau des bouts des doigts, et
- retour vers le cerveau via l'arc sensitif tactile de cette information pour permettre au cerveau de décider l'ajustement de la force de la "pince".

Pour tenter de transmettre néanmoins un tel geste fin, il existe une méthode dite Méthode "Wavetransform", publiée par John Wilson, Neville Hogan du MIT sous le titre "Algorithms for Network - Based Force Feedback", Proceedings of the Fourth PHANTOM Users Group Workshop (PUG 99) Massachusetts Institute of Technology, November 1999. Cette méthode simule le retard introduit par le réseau par une viscosité artificielle qui stabilise la boucle de contre-réaction : le système est d'autant plus visqueux que le réseau introduit un grand retard.

La méthode "Wavetransform" consiste à transposer dans l'espace forces/vitesses la théorie des quadripôles passifs à retard pur qui est bien connue pour les grandeurs électriques tension/intensité. Cette théorie permet de calculer les ondes électriques incidentes et réfléchies en fonction de l'impédance caractéristique de la ligne. La transmission du signal électrique est optimale lorsque cette ligne est fermée sur cette même valeur caractéristique de l'impédance.

La loi d'Ohm U=Z*I se transpose dans l'espace mécanique en la loi F = viscosité * Vitesse et la méthode "Wavetransform" consiste à adapter une ligne à retard pure virtuelle en lui donnant comme impédance (viscosité en fait) caractéristique celle du robot télémanipulé. Le signal est transmis sous la forme de sa transformée en Z, $S(z) = \Sigma (s(t)*e^{(2i*\pi*n*T)})$ où T est le délai fixe du réseau. Plus le réseau introduit un grand retard, et plus il faut rajouter une grande viscosité artificielle dans la ligne pour stabiliser la simulation mécanique répartie du geste fin en boucle fermée et en réseau.

On déforme certes la sensation gestuelle, mais on optimise la transmission du signal utile. Cette méthode a été publiée au Fourth Users Group Workshop (PUG99).

La méthode "Wavetransform" nécessite un réseau synchrone, c'est-à-dire un réseau à retard fixe et connu, par exemple RNIS. Elle est basée sur la représentation en Z des signaux discrets échantillonnés de période égale à ce délai fixe connu du réseau.

Elle est donc inapplicable sur les réseaux asynchrones à messages du type Internet ou ATM, UMTS, qui se caractérisent par un délai de transmission variable, et un rejet si le message se perd ou met trop de temps à traverser le réseau.

Le problème de la cadence trop élevée des systèmes à retours d'efforts est exacerbé sur ces réseaux asynchrones, pour lesquels :

- les messages peuvent se perdre, ne pas aboutir, ou être rejetés si l'accusé de réception tarde trop (TCP/IP),
- les messages qui arrivent à bon port mettent un délai variable pour traverser le réseau,
- ils n'arrivent pas forcément dans l'ordre où ils ont été émis,
- il n'existe pas d'horloge commune exacte à la milliseconde près entre deux machines.

Le document WO 98/06024 décrit un procédé pour fournir un retour de force dans un système comprenant un serveur et un ordinateur comportant une interface homme/machine à retour de force.

Le document FR 2 646 612 décrit un système de jeux de force à distance permettant à deux joueurs éloignés de mesurer leur force sans être physiquement en contact, en s'opposant chacun de leur côté à des équipements électromécaniques raccordés entre eux par un moyen de télécommunications, et restituant à chacun des partenaires les efforts et mouvements effectués par leur adversaire.

Le document « A remote collaboration system using hand-force virtual environment », Makoto Sako et al., Precision and Intelligence Laboratory, Tokyo Institute of Technology, Yokohama, Japan 226, décrit un système de collaboration à distance permettant à des opérateurs situés sur des sites distants de collaborer en maintenant un environnement virtuel mettant en oeuvre des interactions de type « bras de fer » par l'intermédiaire d'un réseau.

L'invention propose de remédier aux inconvénients des systèmes de l'art antérieur.

L'invention propose un système de commande d'un élément à rétroaction situé à distance susceptible de fonctionner avec transmission de données sur réseaux synchrones ou asynchrones, de retard connu ou indéterminé.

Le système de commande, selon un aspect de l'invention, est destiné à un élément à retour d'effort apte à interagir avec un autre élément, du type à constante de temps inférieure à celle liée à une commande à distance. Le système comprend un modèle local pour le calcul d'une consigne destinée à l'élément à retour d'effort à partir d'une variable mesurée par l'élément à retour d'effort, de variables intrinsèques à l'élément à retour d'effort et d'une estimation d'une interaction extérieure sur l'élément à retour d'effort, et d'une variable d'état de l'élément à retour d'effort, un modèle distant pour l'estimation des interactions et des variables d'état de l'autre élément avec mise à jour lors de la réception de données reçues d'un autre système situé à distance, et un moyen de recalage apte à émettre un message de recalage vers ledit autre système.

On effectue ainsi une simulation locale du comportement de l'élément distant.

Avantageusement, le système comprend un modèle fantôme pour effectuer une estimation des variables d'état de l'élément à retour d'effort et recaler ladite estimation à réception du message de recalage. Le modèle fantôme permet de simuler localement le modèle distant du système de commande distant.

Dans un mode de réalisation de l'invention, le moyen de recalage comprend un moyen de comparaison de l'estimation des variables d'état provenant du modèle fantôme et de variables d'état provenant du modèle local de façon qu'en cas de différence supérieure à un seuil prédéterminé, le moyen de recalage émette un message de recalage vers le modèle fantôme et vers l'autre système.

Dans un mode de réalisation de l'invention, le système comprend un moyen d'extrapolation pour traiter un message de recalage provenant de l'autre système et pour mettre à jour le modèle distant.

L'invention propose également un ensemble de commande de deux éléments à retour d'effort situés à distance l'un de l'autre. Chaque élément est pourvu d'un système de commande comprenant un modèle local pour le calcul d'une consigne destinée à l'élément à retour d'effort à partir d'une variable mesurée par l'élément à retour d'effort, de variables intrinsèques à l'élément à retour d'effort et d'une estimation d'une interaction extérieure sur l'élément à retour d'effort, et d'une variable d'état de l'élément à retour d'effort, un modèle distant pour l'estimation des interactions et des variables d'état de l'autre élément avec mise à jour lors de la réception de données reçues d'un autre système situé à distance, et un moyen de recalage apte à émettre un message de recalage vers ledit autre système.

L'invention propose également un procédé de commande d'un élément à retour d'effort apte à interagir avec un autre élément, dans lequel on effectue une modélisation locale pour obtenir une consigne destinée à l'élément à retour d'effort à partir d'une variable mesurée par ledit élément à retour d'effort, de variables intrinsèques audit élément à retour d'effort, d'une estimation d'une interaction extérieure sur l'élément à retour d'effort, et d'une variable d'état de l'élément à retour d'effort; on effectue une modélisation distante des interactions et des variables d'état de l'autre élément avec mise à jour lors de la réception de données reçues d'un autre système situé à distance; on élabore et on émet vers ledit autre système un message de recalage.

Avantageusement, on effectue une modélisation fantôme des variables d'état de l'élément à retour d'effort avec recalage à réception du message de recalage.

Dans un mode de réalisation de l'invention, lors du recalage, on compare l'estimation de variables d'état issues de la modélisation fantôme et de variables d'état issues de la modélisation locale de façon qu'en cas de différence supérieure à un seuil prédéterminé, on émette un message de recalage en vue d'une nouvelle modélisation fantôme, et vers l'autre système.

Dans un mode de réalisation de l'invention, on effectue une extrapolation pour traiter un message de recalage provenant de l'autre système et effectuer une mise à jour de la modélisation distante.

L'invention concerne également un programme d'ordinateur comprenant des moyens de code programme pour mettre en oeuvre les étapes du procédé, lorsque ledit programme fonctionne sur un ordinateur.

L'invention concerne également un support capable d'être lu par un dispositif de lecture de moyens de code programme qui s'y trouvent stockés et qui sont aptes à la mise en oeuvre des étapes du procédé, lorsque ledit programme fonctionne sur un ordinateur.

La présente invention s'applique de façon avantageuse à des systèmes bidirectionnels, par exemple des jeux vidéo avec poignées de commande pourvues d'actionneurs pour le retour d'effort, à la télé-échographie robotisée qui peut être utilisée dans le domaine de l'obstétrique et des examens abdominaux.

Dans le cas de l'échographie, la peau du patient ou de la patiente est généralement enduite d'un gel pour une transmission convenable des ultra sons. La sonde échographique pourra être manipulée à distance par un opérateur. En raison de la présence du gel, les composantes de force exercée par le patient ou la patiente sur la sonde peuvent être considérées comme normales à la surface locale de la peau. La sonde possède 6 degrés de liberté avec une contre réaction de force selon les trois axes d'un repère tridimentionnel et une contre réaction de couple également selon les trois axes d'un repère tridimensionnel. Le système peut également s'appliquer à des jeux de combat à distance, d'escrime, ou encore à des applications industrielles du genre télé-usinage.

La présente invention sera mieux comprise et d'autres avantages apparaîtront à la lecture de la description détaillée de quelques modes de réalisation pris à titre d'exemple nullement limitatifs et illustrés par les dessins annexés, sur lesquels :

la figure 1 est une vue schématique d'un système selon un premier mode de réalisation de l'invention.
la figure 2 est une vue détaillée de la figure 1.
la figure 3 montre les courbes d'évolution de certains paramètres du système.
la figure 4 est une vue schématique d'un second mode de réalisation de l'invention.

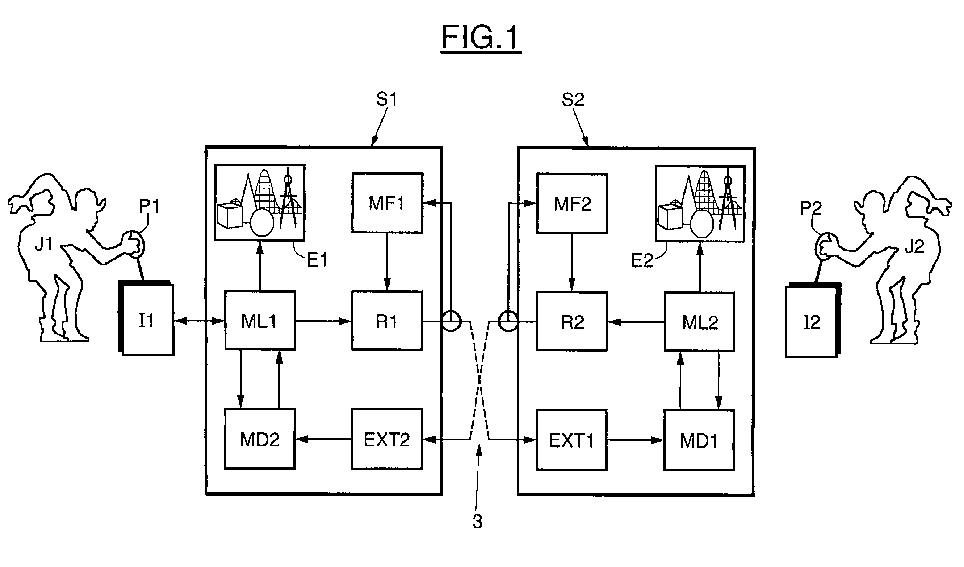

Comme on peut le voir sur la figure 1, un ensemble de jeux où des joueurs J1 et J2 comparent leur force à distance - jeu souvent appelé "bras de fer" - comprend une poignée P1 pour le joueur J1 et une poignée P2 pour le joueur J2. Chaque poignée P1, P2 est reliée à une interface I1, I2 comprenant un moyen pour exercer une force sur la poignée P1, P2, par exemple un actionneur du genre vérin électrique, et un moyen pour mesurer la force exercée par le jour J1, J2 sur la poignée P1, P2, par exemple un capteur de couple ou encore une jauge de contrainte. L'interface I1, I2 comprendra également une carte d'acquisition reliée au moyen d'exercice d'une force et au moyen de mesure et capable d'échanger des données numériques avec un autre système numérique tel qu'un ordinateur.

Chaque interface I1, I2 est reliée à un système de commande S1, S2. Dans le cas illustré ici, les systèmes S1 et S2 sont identiques. Seul le système S1 sera décrit. Toutefois, on peut envisager des modes de réalisation dans lesquels l'un des deux systèmes est de structure simplifiée par rapport à l'autre.

De façon générale, le système S1 peut se présenter sous la forme d'un ordinateur, du genre ordinateur personnel généralement pourvu d'au moins un micro-processeur, de mémoires rémanente et non rémanente, d'un bus de communication, de ports d'entrée et de sortie et d'un ou plusieurs logiciels stockés en mémoire et aptes à être exécutés par le micro-processeur.

Le système S1 est relié d'une part à l'interface I1 par exemple par un bus de type RS 232 et au système S2 par un réseau de communication référencé 3 dans son ensemble et qui pourra être de type synchrone par exemple RNIS ou asynchrone, de type ATM, UMTS ou encore Internet (TCP/IP). Le système S1 est situé à proximité du joueur J1, par exemple dans la même pièce. Le système S2 est situé à distance du système S1, distance qui peut aller de quelques mètres à quelques milliers de kilomètres. En d'autres termes, le système S1, l'interface I1, la poignée P1 et le joueur J1 sont disposés de façon locale tandis que le système S2, l'interface I2, la poignée P2 et le joueur J2 sont disposés de façon distale par rapport aux précédents.

Plus précisément, le système S1 comprend un modèle local ML1 apte à envoyer une consigne à l'interface I1 et à recevoir de ladite interface II une variable mesurée par l'interface I1, par exemple la position X de la poignée P1. La consigne peut être une variable de force ou de couple et est notée $F_e$. Le système S1 comprend un modèle distant MD2 prévu pour estimer un état du modèle local ML2 du système S2. Le modèle distant MD2 du système S1 est apte à recevoir des données en provenance du système S2, à recevoir des données en provenance du modèle local ML 1 et à émettre des données vers le modèle local ML1. Plus particulièrement, le système S1 comprend un extrapolateur EXT2 recevant des données en provenance du système S2 par l'intermédiaire du réseau de communication 3 pour traiter un message de recalage provenant du système S2 et transmettre des données de mise à jour au modèle distant MD2 en fonction du message de recalage reçu en dernier.

Le système S1 comprend un écran E1 relié au modèle local ML1 pour l'affichage de données issues du modèle local ML1, par exemple une courbe retraçant l'évolution des forces exercées et des positions des poignées P1 et P2.

Le système S 1 comprend un recaleur R1 recevant des données du modèle local ML1 et apte à envoyer des données de sortie à destination du système S2, en particulier à destination de l'extrapolateur EXT1 du système S2. Le recaleur R1 est apte à effectuer une préparation de données pour les émettre sous la forme d'un message de recalage qui pourra comprendre une date, la position X de la poignée P1, la force F exercée sur la poignée P 1 à ladite date ainsi que la force exercée sur la poignée P 1 à une date antérieure.

Le système S1 comprend, en outre, un modèle fantôme MF1 qui reçoit également les messages de recalage en provenance du recaleur R1 du système S1 et qui effectue une estimation des variables d'état de l'interface I1 d'après les messages de recalage émis par le recaleur R1 et reçu par le système S2. En d'autres termes, le modèle fantôme MF1 effectue une estimation d'après les mêmes données que celles reçues par le modèle distant MD1 du système S2. Ainsi, le modèle fantôme MF1 permet de modéliser les variables de l'interface I1 telles qu'elles sont modélisées par le système S2.

La sortie du modèle fantôme MF1 est reliée au recaleur R1 qui compare l'estimation des variables d'état provenant du modèle fantôme MF1 et les variables d'état provenant du modèle local ML1. En cas de différence supérieure à un seuil prédéterminé, le recaleur R1 émet un message de recalage destiné au modèle fantôme MF1 et à l'extrapolateur EXT1 du système S2. Ainsi, le volume de données échangé entre les systèmes S1 et S2 est relativement réduit dans la mesure où un message de recalage n'est émis que si l'un des deux systèmes S1, S2 estime que l'autre système S2, S1 n'est plus en mesure d'estimer convenablement ces variables d'état.

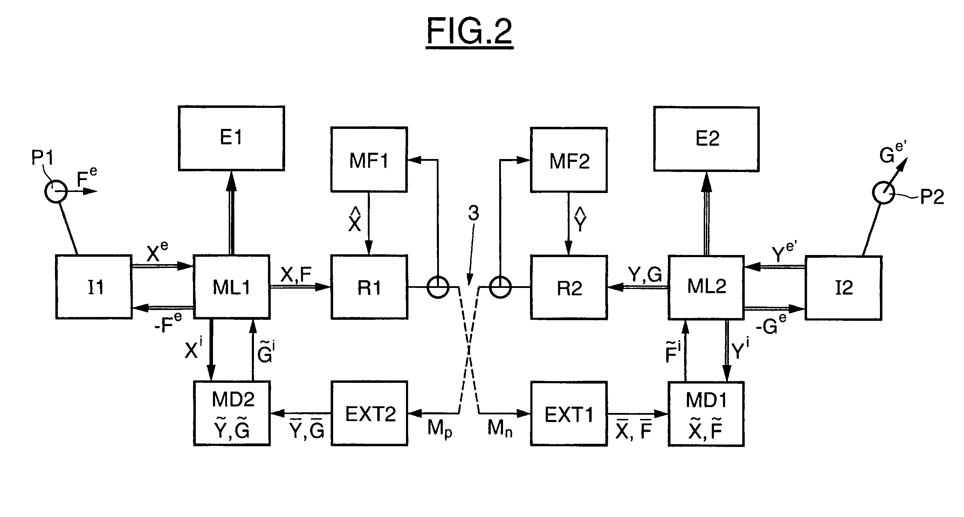

Le fonctionnement du système sera mieux compris en référence à la figure 2. Pour le joueur J1, le vecteur d'état X se décompose en trois parties : $X^e$ variable située à l'interface avec la poignée P1, $X^m$ variable interne au modèle mécanique du joueur J1 et $X^i$ variable d'interaction située à l'interface avec l'autre joueur. De façon analogue, la variable associée de force ou de couple F se décompose en : $F^e$ force exercée par le joueur J1 sur la poignée P1, $F^m$ force exercée par la pesanteur, les autres objets, d'autres joueurs éventuels, et la force $F^i$ exercée par le joueur J1 sur le joueur J2. De manière analogue, le vecteur d'état Y du joueur J2 se décompose en $Y'^e$, $Y'^m$ et $Y'^i$ et le vecteur associé de force de couple G se décompose en $G'^e$, $G'^m$, et $G'^i$. Les deux joueurs J1 et J2 sont en contact virtuel. On a donc $X^i = Y^i$. La loi de l'action et de la réaction donne : $F^i + G'^i = 0$.

A chaque pas de temps, l'interface I1 capte la position $X^e_n$ et la transmet au modèle local ML1. L'interface I1 reçoit la force de consigne $F^e_n$ en provenance du modèle local ML1 et commande son ou ses actionneurs avec la force de contre-réaction $-F^e_n$. De façon analogue, l'interface I2 capte la position $Y'^e_n$ et la transmet au modèle local ML2 et reçoit la force $G'^e_n$ en provenance du modèle local ML2 commande son ou ses actionneurs avec le force de contre-réaction $-G'^e_n$.

Au début de l'instant n +1, le modèle local ML1 reçoit la position $X^e_{n+1}$ de l'interface I1, l'estimation d'interaction $\tilde{G}^i_{n+1}$ du modèle distant MD2 et les variables intrinsèques préenregistrées $F^m_{n+1}$. Le modèle local ML1 calcule la force exercée par le joueur J1 sur le joueur J2: $F^i_{n+1} = \tilde{G}^i_{n+1},$ la force exercée par le joueur J1 sur la poignée P1 :

$$F^e_{n+1} = B^{ee-1} \{ X^e_{n+1} - X^e_n - A^e X_n - B^{em} F^m_{n+1} + B^{ei} \tilde{G}^i_{n+1} \},$$ les matrices A et B étant celles de l'évolution du joueur J1 avec $X$ = AX +BF. Le modèle local ML1 calcule encore :

$$X^{m,i}_{n+1} = X^{m,i}_{n} + A^{m,i} X_{n} + B^{m,i} \begin{bmatrix} F^e_{n+1} \\ F^m_{n+1} \\ -\tilde{G}_{n+1} \end{bmatrix}$$

Le modèle local ML1 envoie $X_{n+1}$ et $F_{n+1}$ au recaleur R1, la consigne - $F^e_{n+1}$ à l'interface I1 et la variable de position $X^i_{n+1}$ au modèle distant MD2.

Le modèle fantôme MF1 s'il ne reçoit pas de message du recaleur R1 calcule $\hat{F}_{n+1} = \hat{F}_{n} + K_1$ , $K_1$ étant fourni par le système S2, et l'estimation de position $\hat{X}_{n+1} = (I+A)\hat{X}_{n} + BF_{n+1}$, c'est-à-dire l'état mécanique du joueur J1 tel qu'il peut être prédit par le système S2. I est ici la matrice identité.

Sur réception d'un message de recalage $M_n = \{n, \overline{X}_n, \overline{F}_n$ et $\overline{F}_{n-1}\}$ en provenance du recaleur R1, le modèle fantôme MF1 effectue le recalage suivant : $\hat{X}_n = \overline{X}_n$, $\hat{F}_n = \overline{F}_n$ et $K1 = \overline{F}_n - \overline{F}_{n-1}$

Le recaleur R1 reçoit à chaque pas de temps n la variable de position $X_n$ et les variables d'effort $F_n$ et $F_{n+1}$ en provenance du modèle local ML1, et l'estimation $\hat{X}_n$ en provenance du modèle fantôme MF1. Il compare la valeur absolue de la différence entre la variable de position $X_n$ et l'estimation $\hat{X}_n$ à un seuil prédéterminé et ne fait rien si ladite valeur absolue est inférieure audit seuil. Dans le cas contraire, il compose un message de recalage $M_n = \{n, X_n, F_n, F_{n-1}\}$. Le recaleur R1 envoie le message de recalage $M_n$ au modèle fantôme MF1 pour qu'il se recale immédiatement et au modèle distant MD1 par l'intermédiaire de l'extrapolateur EXT1 du système S2 pour qu'il se recale le plus tôt possible.

L'extrapolateur EXT2 du système S1 permet de réaliser une synchronisation. En effet, le message $M_p = \{p, Y_p, G_p, G_{p-1}\}$ émis par le recaleur R2 du système S2 arrive au système S1 à un instant compris entre n et n+ 1. Toutefois, le message $M_p$ est estampillé par la date p en provenance du système S2. L'extrapolateur EXT2 calcule $K_2 = G_p - G_{p-1}$ et recale le modèle distant MD2 en effectuant : $\overline{G}_p = G_p$ et $\overline{Y}_p = Y_p$ puis aux instants suivants et quel que soit : j = p,.... n, $\overline{G}_{j+1} = \overline{G}_j + K2$ et $\overline{Y}_{j+1} = \overline{Y}_j + C\overline{Y}_j + D\overline{G}_{j+1}$, C et D étant les matrices équivalentes aux matrices A et B pour le joueur J2. L'extrapolateur EXT2 transmet au modèle distant MD2 le résultat du recalage : $\overline{Y}_{n+1}$, $\overline{G}_{n+1}$, et $K_2$.

Le modèle distant MD2 du système S1 se recale sur réception d'un message en provenance de l'extrapolateur EXT2 en prenant les valeurs fournies par ledit extrapolateur EXT2 :

$$\tilde{G}_{n+1} = \overline{G}_{n+1}, \ \tilde{Y}_{n+1} = \overline{Y}_{n+1} \quad \text{et} \quad \tilde{K}_2 = K_2$$

Hors réception d'un tel message, et à chaque pas de temps, le modèle distant MD2 reçoit la variable de position $X^i_{n+1}$ en provenance du modèle local ML1 et effectue un calcul prédictif :

$$\tilde{G}^{e',m'}_{n+1} = \tilde{G}^{e',m'}_{n} \tilde{K}^{e',m'}2$$

$$\tilde{G}^{i}_{n+1} = D^{ii-1}\left\{ X^i_{n+1} - \tilde{Y}^i_{n} - C\tilde{Y}^i_{n} - D^{e'}G^{e'}_{n+1} - D^{m'}G^{m'}_{n+1} \right\}$$

$$\tilde{Y}^{e',m'}_{n+1} = \tilde{Y}^{e',m'}_{n} + C^{e',m'}\tilde{Y}_{n} + D^{e',m'}\tilde{G}_{n+1}$$

$$\tilde{Y}^{i}_{n+1} = X^i_{n+1}$$

Le modèle distant MD2 transmet au modèle local ML1 la prédiction de variable de position relative au joueur J2 : $\tilde{G}_{n+1}$

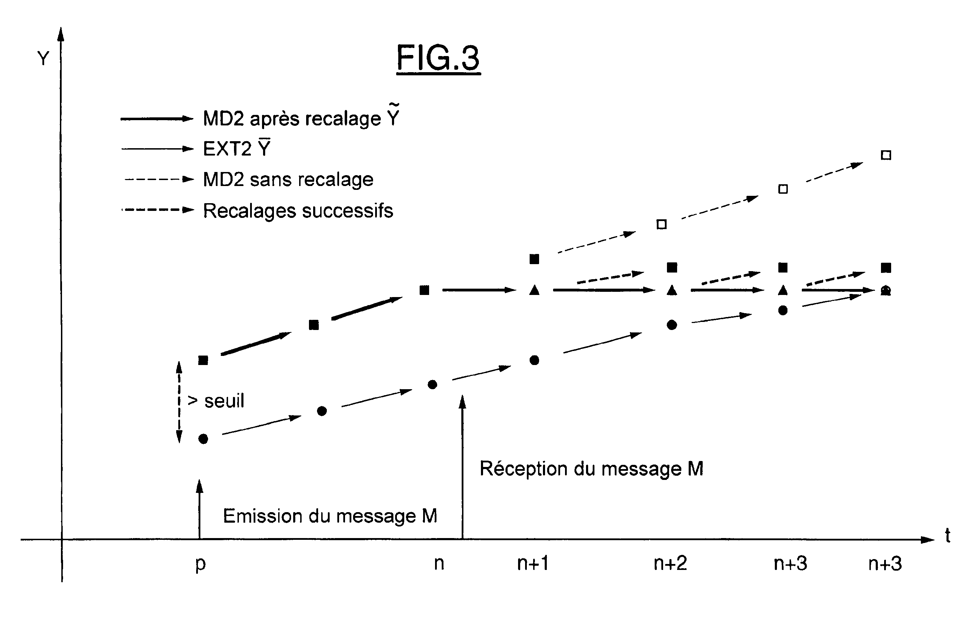

De préférence, l'extrapolateur EXT2 effectue un recalage en biseau qui permet de lisser les évolutions. Un exemple de ce recalage est montré sur les courbes de la figure 3. Au lieu de ramener brutalement l'estimation de la variable de position Y à la variable $\overline{Y}$ calculée comme exposée ci-dessus par l'extrapolateur EXT2, le recalage est effectué en quatre étapes entre les instants n et n+4 selon le calcul suivant :

$$k = \mid \tilde{Y}_{n+1} - \overline{Y}_{n+1} \mid /\text{seuil} + 1 \ ;$$

Si k = 1, alors $\tilde{Y}_{n+1} := \overline{Y}_{n+1}$
Sinon j = n,

$$\overline{G}_{j+1} = \overline{G}_j + K2$$

$$\overline{Y}_{j+1} = \overline{Y}_j + C\overline{Y}_j + D\overline{G}_{j+1}$$

$$\tilde{Y}_{j+1} := \tilde{Y}_j + C\tilde{Y}_j + D\overline{G}_{j+1}$$

$$\tilde{Y}_{j+1} = \left(\overline{Y}_{j+1} + (K-1)\tilde{Y}_{j+1}\right)/k$$

$$j := j+1$$

Si k est supérieur à 2, alors k : = k-1

Sinon on sort de la boucle et $\tilde{Y}_{j+1} = \overline{Y}_{j+1}$. Le recalage en biseau permet un fonctionnement plus doux du système, ce qui est mieux perçu par les utilisateurs et implique moins de contraintes mécaniques.

De façon plus générale, le modèle fantôme MF 1 reçoit les mêmes données que le modèle distant MD1 de l'autre système et permet d'effectuer la même simulation que ledit autre système. En d'autres termes, on cherche à savoir ce que l'autre système ne sait pas dans un but de recalage. Le recaleur fonctionne en aveugle par rapport à l'autre système et permet de continuer à simuler en l'absence de données pertinentes transmises par un message de recalage en provenance de l'autre système. L'extrapolateur EXT2, en particulier dans le cas du recalage en biseau, permet de tenir compte du mouvement tel que mesuré par l'autre système pendant le délai de transmission dû au réseau de communication. Dans une variante simplifiée, on peut parfaitement concevoir que l'un des deux ou les deux systèmes est dépourvu de modèle fantôme. On peut également prévoir de faire fonctionner ensemble un nombre de systèmes supérieur à deux.

Les modèles locaux représentent les modèles mécaniques des deux utilisateurs. Les modèles distants représentent une réplication distante des modèles mécaniques locaux nécessairement approchée du fait des délais de transmission via le réseau de communication des états des modèles locaux. Les modèles fantômes représentent une copie locale approchée des modèles distants. Les modèles distants et les modèles fantômes fonctionnent tous en mode prédicteur-correcteur. Les extrapolateurs effectuent une extrapolation des messages reçus avec un certain retard pour recaler les modèles distants à la valeur de l'horloge de l'autre système. Les recaleurs évaluent la nécessité de lancer un message de recalage sur le réseau de communication dès qu'un écart trop grand apparaît entre les modèles locaux et les modèles fantômes témoins prédictifs locaux des modèles prédictifs distants. Les recaleurs permettent de limiter le nombre de messages émis à travers le réseau de communication pour éviter de l'encombrer. A l'intérieur d'un système, les échanges d'information peuvent être effectués à la cadence d'un khz. Entre les systèmes et donc par l'intermédiaire du réseau de communication, les échanges de message s'effectuent si l'un des recaleurs le considère comme nécessaire.

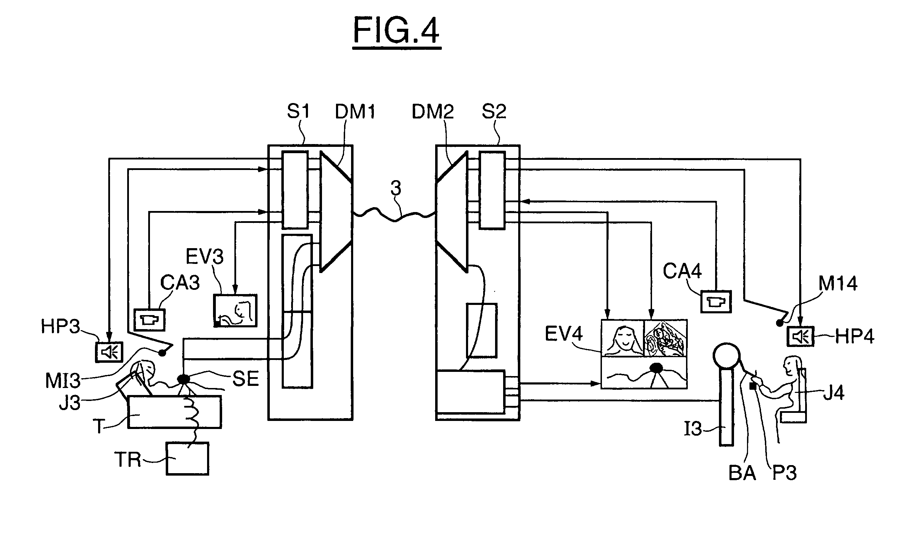

Un mode de réalisation de l'invention destiné à l'échographie est illustré sur la figure 4. Il est prévu un système de commande S1 installé par exemple dans un établissement non spécialisé en obstétrique, dans un établissement d'une ville de petite taille, ou encore dans un véhicule pour la desserte de zones rurales. Le système S2 est installé dans un établissement hospitalier spécialisé où des opérateurs hautement qualifiés sont disponibles pour réaliser les opérations d'échographie, par exemple dans un centre hospitalier régional ou universitaire. Une patiente J3 repose sur un lit ou une table T. Une sonde échographique SE est en contact avec son abdomen. Un tableau de réglage TR de paramètres de la sonde SE est installé à proximité. La sonde SE est relié au système S1 et transmet des données d'images échographiques audit système S1, et échange des données relatives à la position et aux effets exercés avec le système S1. Pour des raisons de clarté du dessin, le support de la sonde SE qui pourrait être un bras articulé n'a pas été représenté ici. Toutefois, on comprend qu'il s'agit d'un support permettant un déplacement dans l'espace selon plusieurs degrés de liberté, en général au moins six pour pouvoir prendre une position adaptée en contact avec l'abdomen de la patiente J3. Il est prévu un microphone MI3 et un haut-parleur HP3 relié au système S1 et permettant à la patiente de converser avec l'opérateur situé à distance. Il est encore prévu une caméra CA3 orientée vers la patiente J3 et un écran vidéo EV3 permettant à la patiente de voir soit l'opérateur situé à distance, soit des images échographiques. La caméra CA3 et l'écran vidéo EV3 sont également reliés au système S1. Les systèmes S 1 et S2, outre les éléments qui ont été décrits en référence aux figures 1 et 2, comprennent chacun un multiplexeur-démultiplexeur DM1 et DM2 pour permettre la transmission de données sur le réseau 3 qui peut être par exemple de type ADSL.

Du côté du système S2, l'opérateur J4 qui pourra être un médecin spécialisé en échographie manipule une poignée P3 dont la position dans l'espace va être répliquée par la sonde SE. La poignée P3 est reliée à un bras articulé BA lui-même relié à une interface I3 du genre des interfaces I1 et I2 décrites ci-dessus et comprenant un ou plusieurs actionneurs et un ou plusieurs capteurs de position et capteurs d'effort. La mesure de l'effet peut être effectuée par une mesure d'une grandeur énergétique des actionneurs, par exemple par le courant consommé encore au moyen d'une jauge de contrainte. L'interface I3 est relié au système S2.

Il est encore prévu une caméra CA4 dirigée vers l'opérateur J4 et dont les images pourront être affichées sur l'écran EV3, un microphone MI4 et un haut-parleur HP4 permettant à l'opérateur J4 de converser avec la patiente J3. Ces éléments sont reliés au système S2. Un écran vidéo EV4 de grande dimension permettra d'afficher simultanément une pluralité d'images, par exemple une image échographique, une image du visage de la patiente J3 et une image montrant le positionnement de la sonde SE sur l'abdomen de la patiente.

## Revendications

**1.** Système de commande (S1) d'un élément à retour d'effort (I1) apte à interagir avec un autre élément (12) commandé par un autre système (S2) situé à distance et apte à communiquer avec ledit système de commande, du type à constante de temps inférieure à celle liée à une commande à distance, ledit systeme de commande étant **caractérisé par le fait par le fait qu'**il comprend un modèle local (ML1) pour le calcul d'une consigne ($F_e$) destinée à l'élément à retour d'effort à partir d'une variable ($X^e$) mesurée par l'élément à retour d'effort, de variables intrinsèques ($F^m$) à l'élément à retour d'effort, d'une estimation ($\tilde{G}$) d'une interaction extérieure sur l'élément à retour d'effort, et d'une variable d'état ($X^{m,i}$) de l'élément à retour d'effort, un modèle distant (MD2) pour l'estimation des interactions et des variables d'état de l'autre élément avec mise à jour lors de la réception de données reçues de l'autre système (S2) situé à distance, et un moyen de recalage (R1) apte à émettre un message de recalage ($M_n$) vers ledit autre système.

**2.** Système selon la revendication 1, **caractérisé par le fait qu'**il comprend un modèle fantôme (MF1) pour effectuer une estimation ($\hat{X},\hat{F}$) des variables d'état de l'élément à retour d'effort et recaler ladite estimation à réception du message de recalage.

**3.** Système selon la revendication 2, **caractérisé par le fait que** le moyen de recalage (R1) comprend un moyen de comparaison de l'estimation des variables d'état provenant du modèle fantôme et de variables d'état provenant du modèle local de façon qu'en cas de différence supérieure à un seuil prédéterminé, le moyen de recalage émette un message de recalage vers le modèle fantôme (MF1) et vers l'autre système (S2).

**4.** Système selon l'une quelconque des revendications précédentes, **caractérisé par le fait qu'**il comprend un moyen d'extrapolation (EXT 2) pour traiter un message de recalage provenant de l'autre système et pour mettre à jour le modèle distant.

**5.** Ensemble de commande de deux éléments distants, chaque élément étant pourvu d'un système de commande selon l'une quelconque des revendications précédentes.

**6.** Procédé utilisé dans un système (S1) de commande d'un élément à retour d'effort (I1) apte à interagir avec un autre élément (I2) commandé par un autre système (S2) situé à distance apte à communiquer avec ledit système de commande, ledit procédé étant **caractérisé en ce qu'**il comprend les étapes suivantes :

- on effectue une modélisation locale pour obtenir une consigne destinée à l'élément à retour d'effort à partir d'une variable ($X^e$) mesurée par ledit élément à retour d'effort, de variables intrinsèques ($F^m$) audit élément à retour d'effort, d'une estimation ($\tilde{G}$) d'une interaction extérieure sur l'élément à retour d'effort, et d'une variable d'état ($X^{m,i}$) de l'élément à retour d'effort ;
- on effectue une modélisation distante des interactions et des variables d'état de l'autre élément avec mise à jour lors de la réception de données reçues de l'autre système (S2) situé à distance ;
- on élabore et on émet vers ledit autre système un message de recalage (R1).

**7.** Procédé selon la revendication 6, dans lequel on effectue une modélisation fantôme des variables d'état de l'élément à retour d'effort avec recalage à réception du message de recalage.

**8.** Procédé selon la revendication 7, dans lequel lors du recalage, on compare l'estimation de variables d'état issues de la modélisation fantôme et de variables d'état issues de la modélisation locale de façon qu'en cas de différence supérieure à un seuil prédéterminé, on émette un message de recalage en vue d'une nouvelle modélisation fantôme, et vers l'autre système.

**9.** Procédé selon l'une quelconque des revendications 6 à 8, dans lequel on effectue une extrapolation pour traiter un message de recalage provenant de l'autre système et effectuer une mise à jour de la modélisation distante.

**10.** Programme d'ordinateur comprenant des moyens de code-programme pour mettre en oeuvre les étapes du procédé selon l'une quelconque des revendications 6 à 9, lorsque ledit programme fonctionne sur un ordinateur.

**11.** Support capable d'être lu par un dispositif de lecture de moyens de code-programme qui s'y trouvent stockés et qui sont aptes à la mise en oeuvre des étapes du procédé selon l'une quelconque des revendications 6 à 9, lorsque ledit programme fonctionne sur un ordinateur.

## Claims

**1.** Control system (S1) for a force feedback element (I1) able to interact with another element (I2) controlled by another remotely located system (S2) and able to communicate with said control system, of the type with time constant less than that associated with a remote control, said control system being **characterized by** the fact that it comprises a local model (ML1) for calculating a set point ($F_e$) intended for the force feedback element from a variable ($X^e$) measured by the force feedback element, variables ($F^m$) intrinsic to the force feedback element, an estimation ($\tilde{G}$) of an external interaction on the force feedback element, and a state variable ($X^{m,i}$) of the force feedback element, a remote model (MD2) for estimating the interactions and the state variables of the other element with update on receipt of data received from the other remotely located system (S2), and a realignment means (R1) able to send a realignment message ($M_n$) to said other system.

**2.** System according to Claim 1, **characterized by** the fact that it comprises a phantom model MF1) for making an estimation ($\hat{X}$, $\hat{F}$) of the state variables of the force feedback element and realigning said estimation on receipt of the realignment message.

**3.** System according to Claim 2, **characterized by** the fact that the realignment means (R1) comprises a means of comparing the estimation of the state variables originating from the phantom model and state variables originating from the local model so that, if there is a difference greater than a predetermined threshold, the realignment means sends a realignment message to the phantom model (MF1) and to the other system (S2).

**4.** System according to any one of the preceding claims, **characterized by** the fact that it comprises an extrapolation means (EXT 2) for processing a realignment message originating from the other system and for updating the remote model.

**5.** Assembly for controlling two remote elements, each element being provided with a control system according to any one of the preceding claims.

**6.** Method used in a system (S1) for controlling a force feedback element (I1) able to interact with another element (I2) controlled by another remotely located system (S2) able to communicate with said control system, said method being **characterized in that** it comprises the following steps:

- a local modelling is performed to obtain a set point intended for the force feedback element from a variable ($X^e$) measured by said force feedback element, variables ($F^m$) intrinsic to said force feedback element, an estimation (G) of an external interaction on the force feedback element, and a state variable ($X^{m,i}$) of the force feedback element;

- a remote modelling is performed of the interactions and the state variables of the other element with update on receipt of data received from the other remotely located system (S2);

- a realignment message (R1) is generated and sent to said other system.

**7.** Method according to Claim 6, in which a phantom modelling is performed of the state variables of the force feedback element with realignment on receipt of the realignment message.

**8.** Method according to Claim 7, in which, on the realignment, the estimation of state variables obtained from the phantom modelling and of state variables obtained from the local modelling are compared so that, if there is a difference greater than a predetermined threshold, a realignment message is sent with a view to a new phantom modelling, and to the other system.

**9.** Method according to any one of Claims 6 to 8, in which an extrapolation is performed to process a realignment message originating from the other system and update the remote modelling.

**10.** Computer program comprising program code means for implementing the steps of the method according to any one of Claims 6 to 9, when said program runs on a computer.

**11.** Medium that can be read by a device for reading program code means that are stored therein and that are able to implement the steps of the method according to any one of Claims 6 to 9, when said program runs on a computer.

**Patentansprüche**

**1.** Steuersystem (S1) eines Kraftrückmeldeelements (I1), das zur Interaktion mit einem anderen Element (I2) geeignet ist, welches von einem anderen System (S2) gesteuert ist, das in der Ferne angeordnet ist und dazu geeignet ist, mit dem Steuersystem zu kommunizieren, vom Typ mit geringerer Zeitkonstante als jene, die mit einer Fernsteuerung verbunden ist, wobei das Steuersystem **dadurch gekennzeichnet ist, dass** es ein lokales Modell (ML1) zur Berechnung einer an das Kraftrückmeldeelement gerichteten Anweisung ($F_e$) auf der Grundlage einer vom Kraftrückmeldeelement gemessenen Variablen ($X^e$), intrinsischer Variablen ($F^m$) des Kraftrückmeldeelements, einer Schätzung ($\tilde{G}$) einer externen Interaktion auf das Kraftrückmeldeelement und einer Zustandsvariablen ($X^{m,i}$) des Kraftrückmeldeelements, ein fernes Modell (MD2) zur Schätzung der Interaktionen und der Zustandsvariablen des anderen Elements mit Aktualisierung nach Empfang von vom anderen, in der Ferne angeordneten System (S2) empfangenen Daten sowie ein Rückstellmittel (R1), das dazu geeignet ist, eine Rückstellnachricht ($M_n$) an das andere System zu senden, aufweist.

**2.** System nach Anspruch 1, **dadurch gekennzeichnet, dass** es ein Phantommodell (MF1) aufweist, um eine Schätzung ($\hat{X}, \hat{F}$) der Zustandsvariablen des Kraftrückmeldeelements durchzuführen und die Schätzung bei Empfang der Rückstellnachricht rückzustellen.

**3.** System nach Anspruch 2, **dadurch gekennzeichnet, dass** das Rückstellmittel (R1) ein Mittel zum Vergleichen der Schätzung der Zustandsvariablen, die vom Phantommodell kommen, mit Zustandsvariablen, die vom lokalen Modell kommen, aufweist, sodass im Falle einer Differenz, die größer ist als ein vorbestimmter Schwellenwert, das Rückstellmittel eine Rückstellnachricht an das Phantommodell (MF1) und an das andere System (S2) sendet.

**4.** System nach einem der vorangegangenen Ansprüche, **dadurch gekennzeichnet, dass** es ein Extrapolationsmittel (EXT 2) aufweist, um eine vom anderen System kommende Rückstellnachricht zu verarbeiten und um das ferne Modell zu aktualisieren.

**5.** Steuersatz aus zwei fernen Elementen, wobei jedes Element mit einem Steuersystem nach einem der vorangegangenen Ansprüche ausgestattet ist.

**6.** Verfahren, das in einem Steuersystem (S1) eines Kraftrückmeldeelements (I1), das zur Interaktion mit einem anderen Element (I2) geeignet ist, welches von einem anderen System (S2) gesteuert ist, das in der Ferne angeordnet und dazu geeignet ist, mit dem Steuersystem zu kommunizieren, verwendet wird, wobei das Verfahren **dadurch gekennzeichnet ist, dass** es die folgenden Schritte umfasst:

- das Durchführen einer lokalen Modellierung zum Erhalt einer an das Kraftrückmeldeelement gerichteten Anweisung auf der Grundlage einer vom Kraftrückmeldeelement gemessenen Variablen ($X^e$), intrinsischer Variablen ($F^m$) des Kraftrückmeldeelements, einer Schätzung (G) einer externen Interaktion auf das Kraftrückmeldeelement und einer Zustandsvariablen ($X^{m,i}$) des Kraftrückmeldeelements;
- das Durchführen einer fernen Modellierung der Interaktionen und der Zustandsvariablen des anderen Elements mit Aktualisierung nach Empfang von vom anderen, in der Ferne angeordneten System (S2) empfangenen Daten;
- das Erstellen und das Senden einer Rückstellnachricht (R1) an das andere System.

7. Verfahren nach Anspruch 6, wobei eine Phantommodellierung der Zustandsvariablen des Kraftrückmeldeelements mit Rückstellung bei Empfang der Rückstellnachricht durchgeführt wird.

8. System nach Anspruch 7, wobei bei der Rückstellung die Schätzung von Zustandsvariablen, die sich aus der Phantommodellierung ergeben, mit Zustandsvariablen, die sich aus der lokalen Modellierung ergeben, verglichen wird, sodass im Falle einer Differenz, die größer ist als ein vorbestimmter Schwellenwert, zwecks einer neuen Phantommodellierung eine Rückstellnachricht an das andere System gesendet wird.

9. Verfahren nach einem der Ansprüche 6 bis 8, wobei eine Extrapolation durchgeführt wird, um eine vom anderen System kommende Rückstellnachricht zu verarbeiten und eine Aktualisierung der fernen Modellierung durchzuführen.

10. Computerprogramm, das Programmcodemittel aufweist, um die Schritte des Verfahrens gemäß einem der Ansprüche 6 bis 9 auszuführen, wenn das Programm auf einem Computer läuft.

11. Träger, der von einer Vorrichtung zum Lesen von Programmcodemitteln, die darauf gespeichert und zur Ausführung der Schritte des Verfahrens gemäß einem der Ansprüche 6 bis 9 geeignet sind, wenn das Programm auf einem Computer läuft, lesbar ist.

FIG.1

EP 1 207 448 B1

FIG.2

FIG.3

légende:
- MD2 après recalage $\tilde{Y}$
- EXT2 $\overline{Y}$
- MD2 sans recalage
- Recalages successifs

> seuil

Réception du message M

Emission du message M

p    n    n+1    n+2    n+3    n+3

EP 1 207 448 B1

## FIG.4

EP 1 207 448 B1

**EP 1 207 448 B1**